# EUROPEAN PATENT APPLICATION

(11) **EP 4 792 734 A1**
(43) Date of publication of application: **19.08.2026**
(21) Application number: 26158736.4
(22) Date of filing: 16.02.2026
(51) Int. Cl.: A61B 5/00, G01N 21/21

(54) **METHOD AND APPARATUS FOR QUANTITATIVELY ESTIMATING BIOLOGICAL PIGMENTS**

(30) Priority: 17.02.2025 KR 20250020181; 28.04.2025 KR 20250055105
(71) Applicant: Korea Advanced Institute of Science and Technology, Daejeon 34141 (KR)
(72) Inventor: KIM, Min Hyuk, 34141 Daejeon (KR); HA, Hyunho, 34141 Daejeon (KR)
(74) Representative: Keller Schneider Patent- und Markenanwälte AG

(57) **Abstract**

The present invention relates to a method and an apparatus for quantitatively estimating biological pigments for static or dynamic measurement of biological pigments such as melanin and hemoglobin using polarization images captured for a dynamic biological object, and includes deriving polarization data using polarization images captured for the dynamic biological object, optimizing biological pigment parameters based on the polarization data, and quantitatively estimating biological pigments in a static state or a dynamic state based on the biological pigment parameters optimized on a per-frame basis.

## Description

This application claims priority to Korean Patent Application No. 10-2025-0020181, filed on February 17, 2025, and Korean Patent Application No. 10-2025-0055105, filed on April 28, 2025, in the Korean Intellectual Property Office, the entire disclosures of which are incorporated herein by reference.

### BACKGROUND

### 1. Field of the Invention

The present invention relates to a method and an apparatus for quantitatively estimating biological pigments, and more particularly, to a method and an apparatus for quantitatively estimating static or dynamic states of biological pigments, such as melanin and hemoglobin, using polarization images captured from a dynamic biological object.

### 2. Description of the Related Art

Acquisition and modeling of polarized light reflection and scattering help reveal the shape, structure, and physical characteristics of an object, and have become increasingly important in the field of computer graphics. In order to describe all possible polarization states, polarization-based systems typically capture and process four-dimensional Stokes vectors.

In addition, capturing polarimetric reflectance requires control over both incident light and outgoing light, each represented as a Stokes vector. Accordingly, a reflectance function is often represented by a four-by-four Mueller matrix that links each component of the incident light to its corresponding outgoing component. Due to the complexity of such a matrix, estimating polarization appearance parameters involves high computational cost and requires additional observations under various incident and outgoing directions and polarization states. Ellipsometry is one of the most commonly used techniques for estimating such a matrix, using structured optical measurements to characterize how light interactions affect polarization states.

Existing methods for capturing spatially varying polarimetric reflectance typically rely on strong assumptions in order to make the problem tractable. As a result, such methods remain limited to acquiring polarimetric appearance information from static and opaque objects.

On the other hand, human faces and other biological objects present a particularly challenging case, due to their complex structure and reflectance properties, strong spatially varying subsurface scattering effects, and dynamic characteristics. Capturing polarization information from such dynamic biological objects is therefore difficult using conventional techniques.

Furthermore, conventional approaches for estimating biological pigments generally focus only on estimating pigment concentrations under static conditions, and have limitations in quantitatively estimating either static physiological indicators or dynamic temporal changes of biological pigments.

### [Prior Art Documents]

### [Non-Patent Documents]

(Non-Patent Document 1) Inseung Hwang, Daniel S. Jeon, Adolfo Munoz, Diego Gutierrez, Xin Tong, and Min H. Kim. 2022. Sparse Ellipsometry: Portable Acquisition of Polarimetric SVBRDF and Shape with Unstructured Flash Photography. ACM Transactions on Graphics, Vol. 41, No. 4 (2022), 14 pages.

### SUMMARY

An object of the present invention is to provide a technique capable of quantitatively estimating, using polarization images captured from a dynamic biological object, static physiological indicators or dynamic temporal changes of biological pigments, including oxy-hemoglobin, deoxy-hemoglobin, eumelanin, and pheomelanin, in an outer layer and an inner layer.

However, the technical problems to be solved by the present invention are not limited to the above-described problems, and may be variously extended without departing from the technical spirit and scope of the present invention.

According to an embodiment of the present invention, there is provided a computer-implemented method for quantitatively estimating biological pigments, the method comprising:
acquiring polarization images captured from a dynamic biological object in a single-view or a multi-view manner and deriving polarization data therefrom;
optimizing biological pigment parameters based on the polarization data; and
quantitatively estimating biological pigments in a static or dynamic state based on the biological pigment parameters optimized for each frame.

According to another embodiment of the present invention, there is provided an apparatus for quantitatively estimating biological pigments, the apparatus comprising:
an image processing unit configured to derive polarization data using polarization images captured from a dynamic biological object;
an optimization unit configured to optimize biological pigment parameters based on the polarization data; and
a quantitative estimation unit configured to quantitatively estimate biological pigments in a static or dynamic state based on the biological pigment parameters optimized for each frame.

According to embodiments of the present invention, biological pigment parameters are estimated by integrating spectral information and polarization information based on polarization images captured from a dynamic biological object. Further, by analyzing pigment parameters estimated for each frame in a temporal sequence, it is possible to quantitatively estimate temporal dynamic changes or static physiological indicators of biological pigments associated with physiological responses such as blood flow recovery and oxygen supply.

However, the advantageous effects of the present invention are not limited to those described above, and may be variously extended without departing from the technical spirit and scope of the present invention.

### BRIEF DESCRIPTION OF THE DRAWINGS

FIG. 1 illustrates polarization image acquisition and processing according to an embodiment of the present invention.
FIG. 2 is a flowchart illustrating an operation flow of a method for quantitatively estimating biological pigments according to an embodiment of the present invention.
FIG. 3 is a block diagram illustrating a detailed configuration of an apparatus for quantitatively estimating biological pigments according to an embodiment of the present invention.
FIGS. 4A to 4F illustrate images related to a multispectral polarimetric imaging system according to an embodiment of the present invention.
FIG. 5 illustrates a graph related to multispectral channel calibration according to an embodiment of the present invention.
FIG. 6 illustrates spectral absorption coefficients of respective biophysical components of skin according to an embodiment of the present invention.
FIG. 7 illustrates results of estimating pigment parameters for a dynamic biological object according to an embodiment of the present invention.
FIG. 8 illustrates temporal dynamic changes of pigment parameters obtained by quantitatively analyzing physiological responses of a dynamic biological object over time according to an embodiment of the present invention.

### DETAILED DESCRIPTION

Advantages and features of the present invention, and methods for achieving the same, will become apparent with reference to embodiments described in detail below in conjunction with the accompanying drawings. However, the present invention is not limited to the embodiments disclosed herein and may be implemented in various different forms. The embodiments are provided to make the disclosure of the present invention complete and to fully convey the scope of the invention to those skilled in the art. The present invention is defined only by the scope of the appended claims.

Unless otherwise defined, all terms including technical and scientific terms used herein have the same meanings as commonly understood by those skilled in the art to which the present invention pertains. Terms defined in generally used dictionaries should be interpreted as having meanings consistent with their meanings in the context of the relevant art, and should not be interpreted in an idealized or overly formal sense unless expressly defined herein.

Hereinafter, embodiments of the present invention will be described in detail with reference to the accompanying drawings. In the drawings, the same reference numerals refer to the same or corresponding elements, and repeated descriptions thereof will be omitted for clarity.

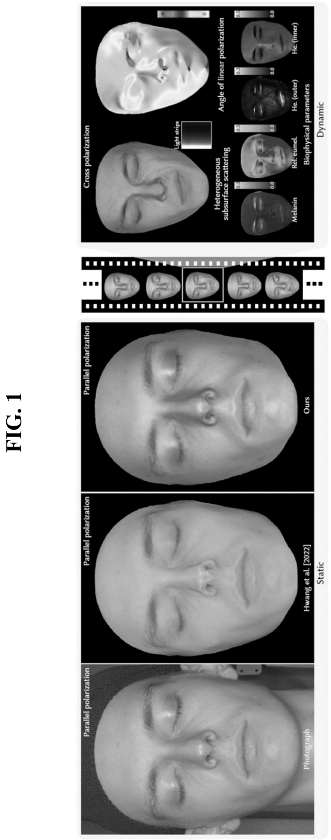

FIG. 1 illustrates polarization image acquisition and processing according to an embodiment of the present invention.

Referring to FIG. 1, the present invention targets a dynamic biological object, that is, a deformable and translucent human face, and illustrates a process of acquiring polarization images, which are polarized multispectral images, from the dynamic biological object, estimating biological parameters such as pigments, and analyzing the same over time.

More specifically, FIG. 1 compares two polarization rendering results, namely a state-of-the-art method (Non-Patent Document 1) and a method according to an embodiment of the present invention (ours), and visually illustrates an overall process from data acquisition to final face (dynamic biological object) rendering, while providing detailed information on what data are collected and processed at each stage.

By considering heterogeneous subsurface scattering, the model according to the embodiment of the present invention can produce more accurate results that are closer to photographic references.

Estimating such subsurface scattering is difficult because light scatters through multiple translucent layers of skin.

The embodiments are based on a subsurface scattering visual model using biophysical pigment parameters, particularly melanin including eumelanin and pheomelanin, and hemoglobin including oxy-hemoglobin and deoxy-hemoglobin.

The hardware configuration according to the embodiments captures six spectral observations and estimates individual contributions to a final appearance based on unique spectral absorption profiles of such biophysical components.

The algorithm according to an embodiment of the present invention consists of two stages.

First, polarization observations of a static face of a dynamic biological object (a subject) are captured over a wide range of angles, and visual parameters serving as initialization for a next stage are optimized through this process.

In the second stage, for each frame, a similar optimization is performed starting from results of the first stage.

By using this approach, high-quality dynamic geometry for each frame and spatially varying appearance parameters represented as texture maps can be obtained.

The technique according to the embodiments of the present invention is the first to capture polarimetric reflectance of a dynamically deformable object.

The embodiments demonstrate results across a wide range of skin tones and facial expressions, and the polarimetric skin model according to the embodiments is seamlessly integrated into many existing rendering pipelines.

First, related research will be described.

Polarimetric imaging has been widely used in computer graphics.

Passive systems use cameras equipped with polarizers positioned in front of a lens or use image sensors, while active systems integrate both polarized light sources and polarized cameras.

In general, such configurations measure only specific polarization states, such as linear polarization or circular polarization at specific angles.

Other works aim to capture polarimetric appearance across different polarization states.

Non-Patent Document 1 combines a polarization array camera with a polarized flashlight.

However, such approaches are limited to static scenes and do not explicitly consider subsurface scattering.

In contrast, according to the methods of the embodiments of the present invention, polarization information of a dynamic biological object can be captured while including the effects of subsurface scattering.

Numerous methods have been developed to acquire high-quality geometric shapes and appearances of static faces.

Since all of these methods require multiple structured light patterns and/or inputs from various viewpoints, they are unsuitable for dynamic capture.

On the other hand, dynamic face capture methods often use passive illumination to capture images of objects under uniform lighting conditions.

Multi-view camera systems use stereo matching to acquire geometric structures or use tracking in image space.

Monocular single-shot methods, video sequences, or binocular video sequences are used to obtain both geometric information and appearance.

Such approaches generally assume simplified reflectance models of human skin, such as including only diffuse albedo or not taking subsurface scattering into account.

Existing studies have developed passive stereo capture systems to acquire specular reflectance and diffuse albedo.

Unlike the methods according to the embodiments of the present invention, such methods assume a predetermined subsurface scattering profile but require each frame to be processed independently for animation sequences.

Since single-shot input is ill-suited for acquiring human skin, recent studies have turned to learning-based methods using active multi-view lighting systems, which are constrained by training and test datasets that do not describe human face reflectance in a physically based manner.

In contrast, the multispectral polarimetric subsurface scattering model according to the embodiments of the present invention yields approximately meaningful, spatially varying, and time-resolved biophysically based visual parameters for dynamic biological objects.

Existing methods for approximating biophysical parameters of human skin generally rely on simplified models, such as assuming diffuse reflectance, not taking subsurface scattering into account, or not handling dynamic appearance changes.

Existing studies have developed image-based methods that separate spatial patterns of melanin and hemoglobin in human skin through independent component analysis of skin color images.

These models were later extended to account for more complex properties of skin based on multispectral images, RGB diffuse reflectance images, or various lighting conditions.

Some of these methods require precomputed textures for inverse rendering, which may lead to visible discretization artifacts or rely on rendered datasets.

With respect to dynamic models, existing studies have presented methods focused on acquiring simplified hemoglobin maps from cross-polarizing filters, which require multiple captures with subjects repeating identical motions.

Subsequently, a statistically based model of human skin was introduced to capture time-varying effects of aging as skin structure and chromophores change over years.

In contrast, according to the embodiments of the present invention, static or dynamic quantitative measurements of biophysically based components such as oxy-hemoglobin, deoxy-hemoglobin, eumelanin, or pheomelanin, as well as full diffusion profiles, are disambiguated through multispectral observation of subsurface scattering without requiring impractical repeated motions.

In addition, the apparatus according to the embodiments of the present invention is capable of simulating appearance changes occurring within seconds rather than decades.

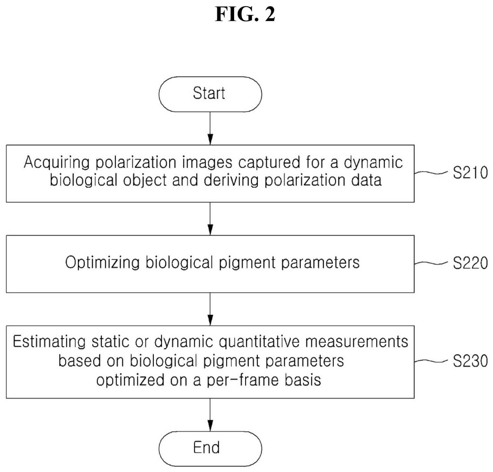

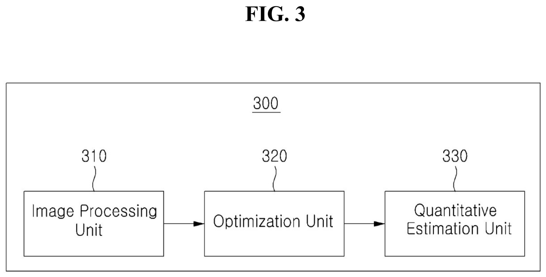

FIG. 2 illustrates an operation flowchart of a method for quantitatively estimating biological pigments according to an embodiment of the present invention, and FIG. 3 illustrates a detailed configuration of an apparatus for quantitatively estimating biological pigments according to an embodiment of the present invention in a block diagram.

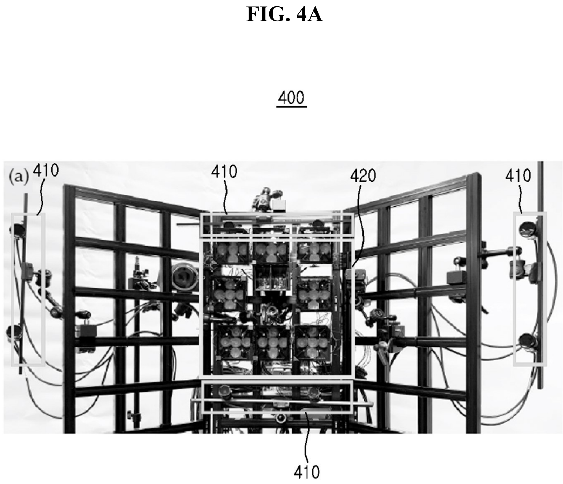

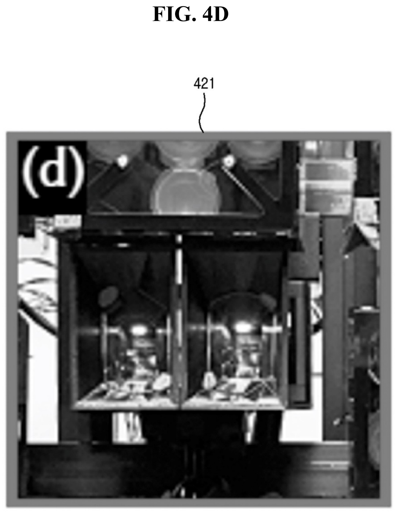

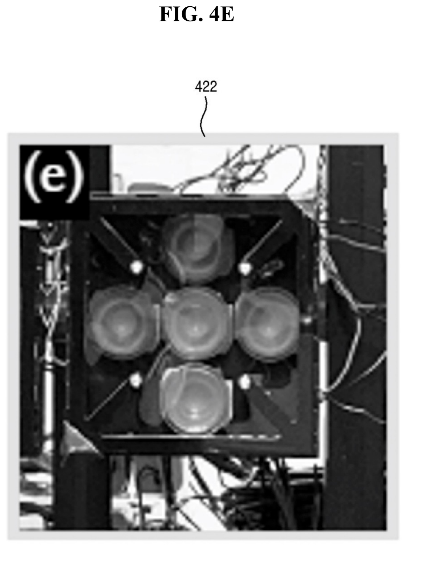

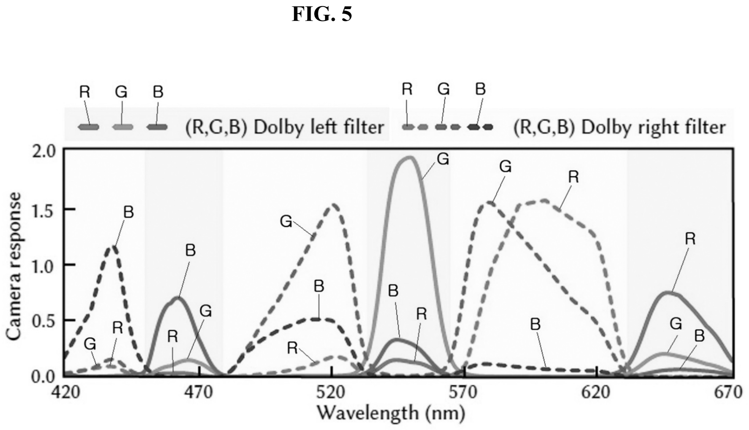

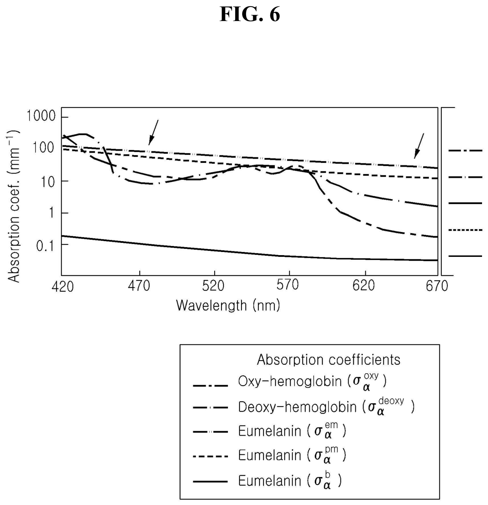

In addition, FIGS. 4A to 4F illustrate images related to a multispectral polarimetric imaging setup according to an embodiment of the present invention, FIG. 5 illustrates a graph related to multispectral-channel calibration according to an embodiment of the present invention, and FIG. 6 illustrates spectral absorption coefficients of respective biophysical components of skin according to an embodiment of the present invention.

Each of the steps illustrated in FIG. 2 (steps S210 to S230) is performed by respective components of an apparatus (300) for quantitatively estimating biological pigments according to an embodiment of the present invention illustrated in FIG. 3, which include an image processing unit (310), an optimization unit (320), and a quantitative estimation unit (330).

Referring to FIGS. 2 and 3, in step S210, the image processing unit (310) acquires polarization images captured from a dynamic biological object.

The polarization images may be acquired as a plurality of polarization image frames captured from a single view or multiple views of the dynamic biological object.

The image processing unit (310) may acquire polarization images captured from the dynamic biological object using a multispectral polarimetric imaging setup.

In this case, the multispectral polarimetric imaging setup is composed of a multispectral central polarimetric module and four additional three-dimensional imaging modules surrounding the central polarimetric module, and the polarimetric three-dimensional imaging module is composed of two polarization machine vision cameras for acquiring dynamic three-dimensional shapes of the dynamic biological object, wherein each of the two polarization machine vision cameras is equipped with three-dimensional glasses and multispectral filters.

Accordingly, the image processing unit (310) may acquire polarization images captured in polarization states of linear polarization or circular polarization from one or more angles with respect to the dynamic biological object using the multispectral polarimetric imaging setup.

Hereinafter, an example of capturing polarization images using the multispectral polarimetric imaging setup will be described with reference to FIGS. 4A to 4F and FIG. 5.

In FIG. 4A, a multispectral polarimetric imaging setup (400) according to an embodiment of the present invention is composed of a multispectral polarimetric module (420) using six multispectral channels together with four three-dimensional stereo imaging modules (410).

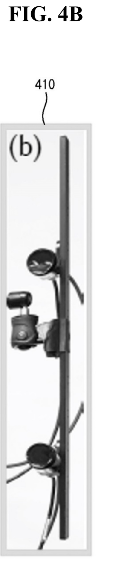

FIG. 4B illustrates a close-up image of the three-dimensional stereo imaging module (410).

The three-dimensional stereo imaging module (410) is composed of two machine vision cameras.

The stereo pair is used to acquire dense depth maps in order to obtain a complete geometric model and corresponding texture mapping for each frame.

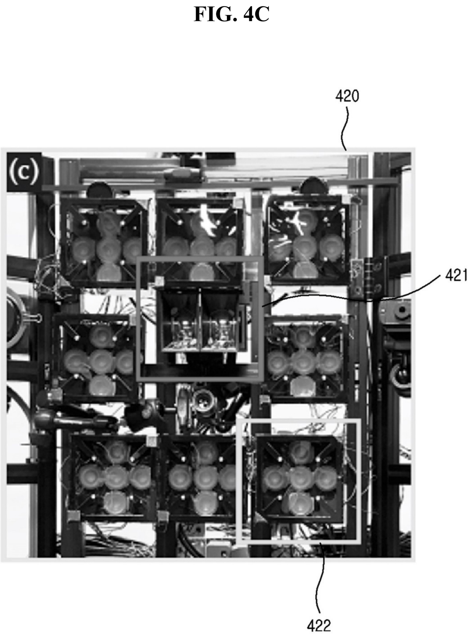

FIGS. 4C to 4E illustrate close-up images of a multispectral polarimetric camera module (422) including two polarization cameras (421) covered with two different Dolby filters and a total of forty LED light sources divided into eight modules, each module being composed of five LEDs.

In the embodiment of the present invention, the multispectral polarimetric module (420) captures a total of six multispectral channels for three RGB channels using two types of Dolby filters.

Each light source is covered with a vertical polarization filter.

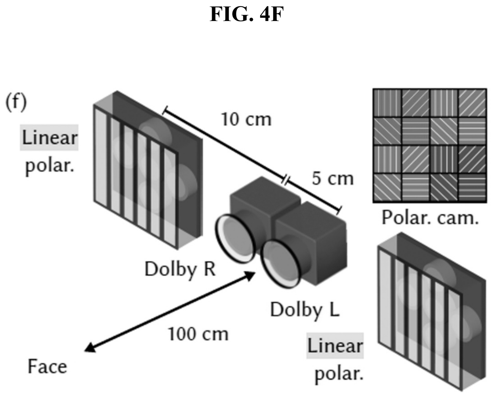

FIG. 4F illustrates a schematic diagram of a configuration of the multispectral polarimetric imaging setup (400) according to an embodiment of the present invention.

The multispectral polarimetric imaging setup (400) also captures four orientations of linear polarization (Linear polar.).

Eight light modules are arranged at intervals of approximately 10 cm, and since the dynamic biological object (Face) is positioned at a distance of approximately 100 cm, the light sources (Light) and the cameras (Polar.cam.) are arranged in a near-coaxial alignment at an angle of approximately 5.72°.

These cameras are synchronized through GPIO cables.

Accordingly, the present invention is capable of capturing images of a dynamic biological object at a frame rate of 20 frames per second (fps).

Referring to FIGS. 4A to 4F, the multispectral polarimetric imaging setup (400) according to an embodiment of the present invention is composed of a multispectral central polarimetric module and four additional three-dimensional imaging modules surrounding the central polarimetric module.

The polarimetric imaging module is composed of two polarization machine vision cameras (BFS-U3-51SPC-C) synchronized at 20 fps, each of which is equipped with off-the-shelf Dolby 3D glasses and different multispectral filters.

Each polarization camera captures four linear polarization components (0°, 45°, 90°, and 135°).

The cameras are surrounded in a near-coaxial configuration by forty linearly polarized LED light sources (CXA-1512) having approximately 1500 lumens and divided into eight modules, each module being composed of five LEDs.

Each Dolby 3D glass further filters wavelength ranges of existing red, green, and blue filters of the camera, thereby effectively halving the range of each channel, wherein the left camera captures an upper half while the right camera captures a lower half, thus generating coverage of the full spectrum with a total of six samples (see FIG. 5).

This is particularly useful for spectral responses of human skin, in which spectral absorption profiles of components are distinguishably different.

Finally, each three-dimensional imaging module is composed of two machine vision cameras for capturing dynamic three-dimensional shapes.

In addition, all cameras are synchronized.

Referring to FIG. 5, left and right glass lenses of Dolby multispectral three-dimensional stereo glasses are disassembled and used as band-pass filters (Dolby left filter and Dolby right filter) in front of two sRGB cameras.

Each color channel is subdivided into two sub-color channels, thereby generating a total of six multispectral channels.

Referring again to FIGS. 2 and 3, in step S210, the image processing unit (310) derives polarization data by calculating observations according to polarization characteristics based on polarization images for each time frame.

The image processing unit (310) may calculate, based on polarization images captured from a plurality of different polarization directions, for each time frame, an unpolarized subsurface scattering observation, a polarized subsurface scattering observation, and a reflection-dominant polarization observation, and derive polarization data including the calculated observations.

More specifically, existing polarization acquisition methods are capable of capturing diffuse and specular information or capturing up to single scattering (Non-Patent Document 1) when assuming that a target object is opaque.

The embodiments of the present invention may introduce a new subsurface scattering ([Equation 1]) in order to handle translucency such as human skin.

Since the light sources and the cameras are arranged in a near-coaxial configuration, sparse ellipsometry algebraic simplification may be applied to a polarimetric BSSRDF model (Non-Patent Document 1).

When polarization images are given from each camera as *I*₀, *I*₉₀, *I*₄₅, and *I*₁₃₅, the optimization unit (330) may calculate the following observations.
- The unpolarized subsurface scattering observation is defined as *I_{SSS}* = 2*I*₉₀ = *S* Σ_{xᵢ}∈*S* ρ*ₛₛₛT⁺⁺*
- The polarized subsurface scattering observation is defined as as *I_{ζ}* = *I*₁₃₅ - *I*₄₅ = *S* Σ_{xᵢ}∈*_{S} ρₛₛₛ T⁻⁺.*
- The reflection-dominant polarization observation is defined as *I_{S} = I*₀ *- I*₉₀ = *S*(*k̅s, ssR⁺* - Σ_{xᵢ}∈*_{S} ρₛₛₛT⁻⁺ζ*).

Here, *k̅s, ss* represents a sum of a specular reflection ks and a single scattering reflection kss. This includes a combination of specular reflection, single scattering, and multiple subsurface scattering.

Here, *R*⁺ = (*R*^{⊥} + *R*^{∥})/2 represents a Fresnel reflection coefficient, *T⁺⁺ = T⁺T⁺* is a product of positive Fresnel transmission coefficients, and *T⁻⁺ = T⁻T⁺* is a product of negative and positive coefficients.

The embodiments may acquire such observations on a per-frame basis and use them as input to a reconstruction algorithm for optimization in order to obtain full dynamic data.

In step S220, the optimization unit (320) optimizes biological pigment parameters based on polarization data for each time frame.

Here, the biological pigment parameters may include quantitative concentrations of oxy-hemoglobin, deoxy-hemoglobin, eumelanin, and pheomelanin.

The optimization unit (320) may optimize biological pigment parameters through an optimization algorithm based on a biophysically based model using polarization data.

At this time, the optimization unit (330) may use the polarization data as input values of an optimization algorithm, which is a coordinate descent method using alternating least squares (ALS), to optimize biological pigment parameters for a skin region of a dynamic biological object for each time frame.

Thereafter, the optimization unit (320) may input, for each time frame, the calculated unpolarized subsurface scattering observation, polarized subsurface scattering observation, and reflection-dominant polarization observation to an optimization algorithm based on a biophysically based model, and may optimize biological pigment parameters for a skin region of the dynamic biological object for each time frame.

Here, the optimization algorithm may quantify concentrations of corresponding pigment parameters based on multispectral absorption profiles of respective pigments, and may be a coordinate descent method using alternating least squares (ALS).

In addition, the optimization algorithm may be performed in a manner that minimizes photometric loss according to subsurface scattering distance within skin using a Gaussian-based diffusion profile.

More specifically, with respect to the biophysically based model, the embodiments of the present invention adopt a two-layer model composed of two layers, each layer being characterized by absorption and reduced scattering coefficients.

Referring to FIG. 6, spectral absorption coefficients of respective biological pigment parameters of skin included in the model (oxy-hemoglobin, deoxy-hemoglobin, eumelanin, pheomelanin, and a skin base layer) are illustrated.

First, absorption of an outer layer will be described.

The (spectral) absorption coefficient of the outer layer, ${σ}_{a}^{out}$*,* is mainly attributed to the presence of melanin in the epidermis and, to a lesser extent, to the presence of hemoglobin in the upper dermis, and may be defined as follows. $\begin{matrix}{σ}_{a}^{out} = {C}_{m} \left({β}_{m}{σ}_{a}^{em}+\left(1-{β}_{m}\right){σ}_{a}^{pm}\right) + {C}_{h , out} \left({γ}_{out}{σ}_{a}^{oxy}+\left(1-{γ}_{out}\right){σ}_{a}^{deoxy}\right) \\ + \left(1-{C}_{m}-{C}_{h , out}\right) {σ}_{a}^{b}\end{matrix}$

Here, *Cₘ* and *C_{h,out}* are fractions of melanin and hemoglobin in the outer layer, respectively, *βₘ* is a fraction of eumelanin in melanin, and *γₒᵤₜ* is an oxy-hemoglobin fraction of hemoglobin. The values of *Cₘ, C_{h,out},* and *βₘ* are estimated from captured data while *γₒᵤₜ* is constant. Different spectral absorption coefficients *σₐ* of eumelanin (em), pheomelanin (pm), oxy-hemoglobin (oxy), deoxy-hemoglobin (deoxy), and base (b) are provided in previous studies.

Next, absorption of the inner layer will be described.

The inner layer is composed of a dense capillary network including hemoglobin in the dermis.

The absorption coefficient of the inner layer, ${σ}_{a}^{in}$*,* is mainly explained by hemoglobin and may be expressed as follows. ${σ}_{a}^{in} = {C}_{h , in} \left({γ}_{in}{σ}_{a}^{oxy}+\left(1-{γ}_{in}\right){σ}_{a}^{deoxy}\right) + \left(1-{C}_{h , in}\right) {σ}_{a}^{b}$

Here, *C_{h,in}* is a fraction of hemoglobin in the inner layer, and *γᵢₙ* is an oxy-hemoglobin fraction. The value of *C_{h,in}* is also estimated from captured data.

The embodiments of the present invention assume that the ratio of oxy-hemoglobin to hemoglobin is the same in the inner layer and the outer layer, and, similar to other existing models, has a fixed value of *γ* = *γᵢₙ* = *γₒᵤₜ* = 0.75.

Next, reduced scattering will be described.

At a wavelength *λₙₘ*, a spectral reduced scattering coefficient of the outer layer, ${σ}_{s}^{out ′}$ may be defined as follows. ${σ}_{s}^{out ′} \left(λ\right) = 14.74 \times {λ}^{- 0.22} + 2.2 {E}^{11} \times {λ}^{- 4}$

The reduced scattering coefficient of the inner layer is 50% of the scattering coefficient of the outer layer, and thus does not need to be explicitly estimated.

In the present invention, setting the reduced scattering coefficient of the inner layer to a predetermined ratio (e.g., approximately 50%) of the scattering coefficient of the outer layer corresponds to one exemplary configuration adopted to improve computational efficiency.

However, such a ratio may vary depending on imaging conditions, physiological characteristics of the target object, wavelength range, camera and light source configuration, or system calibration conditions, and the present invention also encompasses cases in which the inner-layer scattering coefficient is set to a different ratio relative to the outer-layer scattering coefficient or is independently estimated.

In addition, with respect to describing a process of optimizing biological pigment parameters, in order to estimate a full diffusion profile *ρₛₛₛ* from multispectral observations of subsurface scattering *ρ̅ₛₛₛ*, spectral profiles of absorption coefficients of oxy-hemoglobin, deoxy-hemoglobin, eumelanin, pheomelanin, and a skin base parameter are relied upon.

Since absorption coefficients vary according to spectral structure, multispectral measurements (see FIG. 5) are utilized to disambiguate concentrations of various biophysical components of a skin model (*Cₘ*, *βₘ, C_{h,in},* and *C_{h,out}*)*.*
he embodiments minimize photometric loss between *ρ̅ₛₛₛ* and rendered subsurface scattering using the full diffusion profile.

A major challenge is differentiation of the diffusion profile with respect to biophysical parameters, since forward optimization is neither efficient nor scalable for high-resolution textures.

To address the lack of end-to-end derivation, a coordinate descent method using alternating least squares is proposed.

Accordingly, optimization according to embodiments of the present invention is divided into two subproblems.

First, Gaussian weights defining *ρₛₛₛ* are obtained, and second, biophysical parameters of *ρ̅ₛₛₛ* are estimated.

The embodiments discretize spectral absorption into 15 multispectral channels.

To compute photometric loss, these channels are converted into six channels of the camera using a spectral calibration function of the system.

For efficiency, initial-stage iterations are computed at a coarser resolution.

The embodiments approximate each profile using nine Gaussians and render a full appearance model by merging the subsurface scattering with contributions of specular and single scattering components.

Using this efficient approach, biological pigment parameters of skin can be approximated through fast gradient descent iterations.

The coordinate descent approach using alternating least squares (ALS) described in the present invention is one of the preferred embodiments for efficiently estimating biological pigment parameters.

However, the technical concept of the present invention encompasses all forms of optimization techniques that iteratively update biological pigment parameters so as to minimize error between polarization data and a biophysics-based model, including cases using gradient descent, stochastic gradient descent (SGD), modified coordinate descent methods, approximate inference-based methods, or combinations thereof

In step S230, the quantitative estimation unit (330) quantitatively estimates biological pigments in a static or dynamic state based on biological pigment parameters optimized on a per-frame basis.

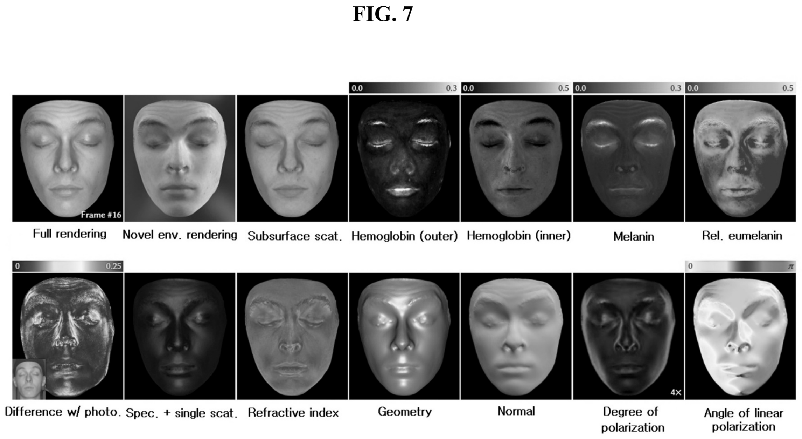

The quantitative estimation unit (330) may estimate static physiological indicators based on biological pigment parameters optimized for each frame, or may quantitatively calculate variations according to time differences based on a time sequence of biological pigment parameters optimized per frame, and may estimate dynamic changes such as blood flow recovery or oxygen supply state according to the calculated results (see FIG. 7).

In the present invention, the quantitative estimation unit (330) is characterized in that it can perform static measurements and, if necessary, can also measure dynamic variations.

In this case, static estimation may indicate quantitatively estimating a concentration or distribution of biological pigments at a corresponding moment based on biological pigment parameters at a specific time point.

In addition, dynamic estimation may indicate quantitatively estimating temporal variations of biological pigments based on biological pigment parameters optimized for successive frames over time.

FIG. 7 illustrates results of estimating pigment parameters for a dynamic biological object according to an embodiment of the present invention.

FIG. 7 illustrates images of full rendering, novel environment rendering, subsurface scattering, hemoglobin (outer), hemoglobin (inner), melanin, relative eumelanin, difference with photo, specular plus single scattering, refractive index, geometry, normal, degree of polarization, and angle of linear polarization for a dynamic biological object based on data of eleven individuals with various skin tones, gender identities, and ethnic backgrounds.

Referring to FIG. 7, result images capturing polarimetric reflectance parameters, biophysical parameters, and geometric structures with high accuracy according to embodiments of the present invention can be confirmed.

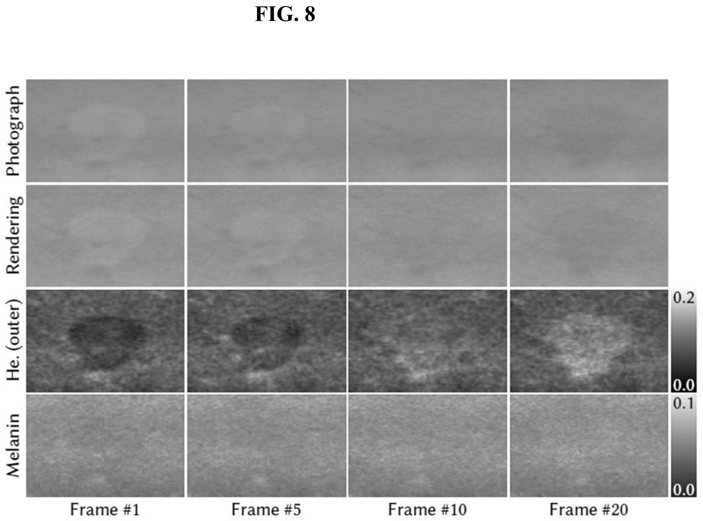

FIG. 8 illustrates dynamic changes in pigment parameters obtained by quantitatively analyzing physiological responses of a dynamic biological object over time according to an embodiment of the present invention.

FIG. 8 illustrates changes in blood flow at a specific region of the dynamic biological object after pressure applied to the region is released.

Referring to FIG. 8, the present invention aligns a plurality of frames consecutively captured for the same region of the dynamic biological object in chronological order, and analyzes a blood flow recovery process after compression by arranging hemoglobin concentrations estimated in each frame as a time series.

Accordingly, when examining FIG. 7 as an analysis result, it can be observed that the hemoglobin concentration in the outer layer returns to a previous level as blood flows back into the compressed region.

As a result, the present invention demonstrates that dynamic changes of pigments according to physiological responses can be quantitatively identified without repetitive motion.

Although the biophysical model described in the present invention has been explained by way of example as a two-layer structure divided into an outer layer and an inner layer, this is merely one embodiment provided to facilitate understanding of the present invention.

The quantitative estimation results of biological pigments according to the present invention may be utilized not only for medical diagnostic purposes, but also for skin condition analysis, biometric signal monitoring, user state recognition, personalized content generation, virtual human rendering, image synthesis, healthcare services, interactive media, and other non-medical applications.

Accordingly, the present invention is not limited to a specific application field and is applicable to all systems and services that utilize quantitative estimation results of biological pigments.

The technical concept of the present invention includes a general framework for modeling skin, biological tissue, or a translucent biological object as one or more layers and estimating absorption, scattering, and polarization characteristics of each layer, and may be applied, as necessary, to a multilayer model including three or more layers, a model having a continuous depth distribution, or a model in which the number of layers is adaptively varied.

Accordingly, the present invention is not limited to a specific number of layers or a specific layer structure, and encompasses various biological models in which biological pigments or biophysical parameters are distributed along a depth direction.

The system or apparatus described above may be implemented using hardware components, software components, or a combination of hardware components and software components.

For example, the apparatuses and components described in the embodiments may be implemented using one or more general-purpose computers or special-purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of executing and responding to instructions.

The processing device may execute an operating system (OS) and one or more software applications executed on the operating system.

In addition, the processing device may access, store, manipulate, process, and generate data in response to execution of software.

For convenience of understanding, although the processing device is described as being singular, those skilled in the art will appreciate that the processing device may include a plurality of processing elements and/or multiple types of processing elements.

For example, the processing device may include a plurality of processors or one processor and one controller.

In addition, other processing configurations, such as a parallel processor, are also possible.

Software may include computer programs, code, instructions, or a combination of one or more thereof, and may configure the processing device to operate as desired or instruct the processing device independently or collectively.

Software and/or data may be permanently or temporarily embodied in any type of machine, component, physical device, virtual equipment, computer storage medium or device, or transmitted signal wave in order to be interpreted by or provide instructions or data to the processing device.

Software may be distributed and stored or executed in a distributed manner on network-connected computer systems.

Software and data may be stored in one or more computer-readable recording media.

The method according to embodiments may be implemented in the form of program instructions executable through various computer means and recorded on a computer-readable medium.

The computer-readable medium may include program instructions, data files, data structures, or a combination thereof.

The program instructions recorded on the medium may be specially designed and configured for the embodiments or may be known and available to those skilled in the art of computer software.

Examples of computer-readable recording media include hardware devices specially configured to store and execute program instructions, such as hard disks, magneto-optical media, solid-state drives (SSDs), read-only memory (ROM), random-access memory (RAM), flash memory, and the like.

Examples of program instructions include not only machine language code generated by a compiler, but also high-level language code executable by a computer using an interpreter or the like.

The hardware devices described above may be configured to operate as one or more software modules in order to perform operations of the embodiments, and vice versa.

Although the embodiments have been described above with reference to limited embodiments and drawings, those skilled in the art will appreciate that various modifications and variations may be made based on the above description.

For example, appropriate results may be achieved even if the described techniques are performed in an order different from the described methods, and/or components of the described systems, structures, apparatuses, circuits, and the like are combined or assembled in a form different from the described methods, or replaced or substituted with other components or equivalents.

Therefore, other implementations, other embodiments, and equivalents to the claims also fall within the scope of the claims described below.

## Claims

1. A computer-implemented method for quantitatively estimating biological pigments, comprising:
deriving polarization data using polarization images captured for a dynamic biological object in a single view or a multi-view at a plurality of viewpoints;
optimizing biological pigment parameters based on the polarization data; and
quantitatively estimating biological pigments in a static state or a dynamic state based on the biological pigment parameters optimized on a per-frame basis.

2. The method of claim 1,
wherein deriving the polarization data using the polarization images comprises
acquiring the polarization images captured in a polarization state of linear polarization or circular polarization at one or more angles for the dynamic biological object using a multispectral polarimetric imaging system.

3. The method of claim 2,
wherein the multispectral polarimetric imaging system
comprises a multispectral module, a central polarimetric module, and four additional 3D imaging modules surrounding the central polarimetric module, and
wherein the polarimetric 3D imaging module
comprises two polarization machine vision cameras for acquiring dynamic 3D geometry of the dynamic biological object,
each of the two polarization machine vision cameras being equipped with 3D glasses and a multispectral filter.

4. The method of claim 1,
wherein deriving the polarization data using the polarization images comprises
calculating, based on the polarization images captured from a plurality of different polarization directions, for each time frame, an unpolarized subsurface scattering observation, a polarized subsurface scattering observation, and a reflection-dominant polarization observation, and deriving the polarization data including the calculated observations.

5. The method of claim 1,
wherein optimizing the biological pigment parameters comprises
applying the polarization data to a biophysically based model and optimizing the biological pigment parameters through an optimization algorithm.

6. The method of claim 5,
wherein optimizing the biological pigment parameters comprises
using the polarization data as input values of an optimization algorithm, which is a coordinate descent method using alternating least squares (ALS), to optimize biological pigment parameters for a skin region of the dynamic biological object for each time frame.

7. The method of claim 6,
wherein the optimization algorithm
quantifies concentrations of corresponding pigment parameters based on multispectral absorption profiles of respective pigments.

8. The method of claim 7,
wherein the optimization algorithm
is performed in a manner that minimizes photometric loss according to subsurface scattering distance within skin using a Gaussian-based diffusion profile.

9. The method of claim 1,
wherein the biological pigment parameters
include quantitative concentrations of oxy-hemoglobin, deoxy-hemoglobin, eumelanin, and pheomelanin.

10. The method of claim 1,
wherein quantitatively estimating the biological pigments comprises
estimating static physiological indicators based on the biological pigment parameters optimized on a per-frame basis, or
quantitatively calculating variations according to time differences based on a time sequence of the biological pigment parameters optimized on a per-frame basis, and estimating dynamic changes including blood flow recovery or oxygen supply state according to calculated results.

11. An apparatus for quantitatively estimating biological pigments, comprising:
an image processing unit configured to derive polarization data using polarization images captured for a dynamic biological object in a single view or a multi-view at a plurality of viewpoints;
an optimization unit configured to optimize biological pigment parameters based on the polarization data; and
a quantitative estimation unit configured to quantitatively estimate biological pigments in a static state or a dynamic state based on the biological pigment parameters optimized on a per-frame basis.

12. The apparatus of claim 11,
wherein the image processing unit
is configured to acquire the polarization images captured in a polarization state of linear polarization or circular polarization at one or more angles for the dynamic biological object using a multispectral polarimetric imaging system.

13. The apparatus of claim 12,
wherein the image processing unit
is configured to calculate, based on the polarization images captured from a plurality of different polarization directions, for each time frame, an unpolarized subsurface scattering observation, a polarized subsurface scattering observation, and a reflection-dominant polarization observation, and to derive the polarization data including the calculated observations.

14. The apparatus of claim 11,
wherein the optimization unit
is configured to use the polarization data as input values of an optimization algorithm, which is a coordinate descent method using alternating least squares (ALS), to optimize biological pigment parameters for a skin region of the dynamic biological object for each time frame.

15. The apparatus of claim 11,
wherein the quantitative estimation unit
is configured to estimate static physiological indicators based on the biological pigment parameters optimized on a per-frame basis, or to quantitatively calculate variations according to time differences based on a time sequence of the biological pigment parameters optimized on a per-frame basis, and to estimate dynamic changes including blood flow recovery or oxygen supply state according to calculated results.
